# EUROPEAN PATENT APPLICATION

(11) **EP 2 106 750 A2**
(43) Date of publication of application: **07.10.2009**
(21) Application number: 09004844.8
(22) Date of filing: 01.04.2009
(51) Int. Cl.: A61B 17/00

(54) **Operation system**

(30) Priority: 02.04.2008 JP 2008096109
(71) Applicant: Olympus Medical Systems Corporation, Tokyo 151-0072 (JP)
(72) Inventor: Sekino, Naomi, Tokyo 151-0072 (JP); Gotanda, Masakazu, Tokyo 151-0072 (JP)
(74) Representative: von Hellfeld, Axel

(57) **Abstract**

An operation system has a controller (2) controlling a plurality of medical devices and a footswitch (3) transmitting an instruction signal to the controller, the footswitch (3) having a selection unit (3c) capable of selecting, from the medical devices, a medical device to be used and an output of the medical device; and a first and second pedals (3a, 3b) capable of instructing the selected medical device to perform the output, and the controller (2) having a memory unit (13) storing output pedal setting information comprising operator identification information identifying an operator; first pedal setting information comprising device identification information identifying the medical device corresponding to the first pedal and information about an output of the medical device corresponding to the first pedal; and second pedal setting information comprising device identification information identifying the medical device corresponding to the second pedal and information about an output of the medical device corresponding to the second pedal; and a control unit (11) controlling, when the selection unit selects given output pedal setting information and the respective pedals are held down, the respective medical devices on the basis of the first and second pedal setting information in the output pedal setting information.

## Description

### Background of the Invention

### Field of the Invention

The present invention relates to the control of medical devices.

### Description of the Related Art

Conventionally, an apparatus for operating medical devices such as an ultrasonic cutting and coagulating system (US) and electric surgical knives (monopolar and bipolar) used in an operation room while sequentially switching the outputs of the devices using a single unit of footswitch has been known (for example, Japanese Laid-open Patent Publication No. 2002-238919). In addition, an ophthalmic operation apparatus with which the functions of the footswitch can be set in accordance with the need of the operator has been disclosed (for example, Japanese Laid-open Patent Publication No. 11-244321), as well as Japanese Laid-open Patent Publication No. 9-276214 and Japanese Laid-open Patent Publication No. 6-78920.

### Summary of the Invention

An operation system according to an embodiment of the present invention has
a controller controlling a plurality of medical devices and
a footswitch transmitting an instruction signal to the controller,
the footswitch having
a selection unit capable of selecting, from the medical devices, a medical device to be used and an output of the medical device; and
a first and second pedals capable of instructing the selected medical device to perform the output, and the controller having
a memory unit storing output pedal setting information comprising operator identification information identifying an operator; first pedal setting information comprising device identification information identifying the medical device corresponding to the first pedal and information about an output of the medical device corresponding to the first pedal; and second pedal setting information comprising device identification information identifying the medical device corresponding to the second pedal and information about an output of the medical device corresponding to the second pedal; and
a control unit controlling, when the selection unit selects given output pedal setting information and the respective pedals are held down, the respective medical devices on the basis of the first and second pedal setting information in the output pedal setting information.

An operation system according to an embodiment of the present invention has
a plurality of medical devices;
a controller controlling the medical devices;
a footswitch having first and second pedals capable of instructing the medical devices to perform the output and transmitting an instruction signal to the controller in accordance with holding-down states of the respective pedals; and
a wristband worn on each arm of the operator, the wristband having an IC tag storing operator identification information of the operator and left/right classification information determining on which arm the wristband is worn,
the medical device having
a reading unit reading the IC rag to obtain the operator identification information and the left/right classification information;
a device-in-use information generation unit generating device-in-use information in which the operator identification information and the left/right classification information are associated with device identification information of the medical device itself; and
a transmission unit transmitting the device-in-use information, and
the controller having
a memory unit storing output pedal setting information comprising operator identification information identifying an operator; first pedal setting information comprising device identification information identifying the medical device corresponding to the first pedal and information about an output of the medical device corresponding to the first pedal; and second pedal setting information comprising device identification information identifying the medical device corresponding to the second pedal and information about an output of the medical device corresponding to the second pedal; and
a control unit searching the output pedal setting information from the memory unit on the basis of the operator ID information in the device-in-use information; extracting, from the output pedal setting information, the output pedal setting information composed of a combination of first pedal setting information and second pedal information corresponding to the left/right classification information and the medical device identification information in the device-in-use information; and controlling the respective medical devices on the basis of the extracted first and second pedal setting information, in accordance with holding-down states of the respective pedals.

### Brief Description of the Drawings

Fig. 1 illustrates a surgical operation system 1 in the first embodiment.
Fig. 2 illustrates the outline of the internal configuration of a controller 2 in the first embodiment.
Fig. 3 illustrates a start menu screen 21 displayed on a display panel 5 in the first embodiment.
Fig. 4 illustrates a course setting screen 31 displayed on a display panel 5 in the first embodiment.
Fig. 5 illustrates a footswitch function assignment setting table 50 in the first embodiment.
Fig. 6 illustrates a step switching screen 61 displayed on a display panel 5 in the first embodiment.
Fig. 7 illustrates a process flow of a step switching operation in the first embodiment.
Fig. 8 illustrates the overall configuration of a surgical operation system 70 in the second embodiment.
Fig. 9 illustrates an example of footswitch usage history information 72 in the second embodiment.
Fig. 10 illustrates processes performed by a computer 71 in the second embodiment.
Fig. 11 illustrates an example of a medical device operation history checking screen in the second embodiment.
Fig. 12 illustrates the overall configuration of a surgical operation system 80 in the third embodiment.
Fig. 13 illustrates the outline of an IC tag reading and transmitting mechanism in a SonoSurg probe 81 in the third embodiment.
Fig. 14 illustrates the transition of data from the acquisition to the transmission, to a reception terminal 85, of IC tag information by an IC tag reading and transmitting mechanism 91 in the third embodiment.
Fig. 15 illustrates a footswitch function assignment setting table 111 in the third embodiment.
Fig. 16 illustrates a flow of a series of processes in the third embodiment.

### Description of the Preferred Embodiments

### <First embodiment>

According to the disclosure of Japanese Laid-open Patent Publication No. 2002-238919, a footswitch has an output mode switching unit, and the mode selection state is displayed overlapping an endoscope image on a monitor. In this case, however, some operators may select, by mistake, an output that is not to be used. In addition, the display of the selected mode overlapping the endoscope image may interfere with the field of view of the operator.

Meanwhile, according to Japanese Laid-open Patent Application No. 11-244321, the respective outputs are switched using a plurality of operation pedals, resulting in a troublesome switching operation and a risk of selecting, by mistake, an output that is not to be used.

Therefore, the present embodiment provides an operation system in which output operations of medical devices required by each operator can be switched by minimum necessary switching operations. Particularly, an operation system in which medical devices to be used can be switched and their outputs can be adjusted using a footswitch having one switching button and two pedals is described below.

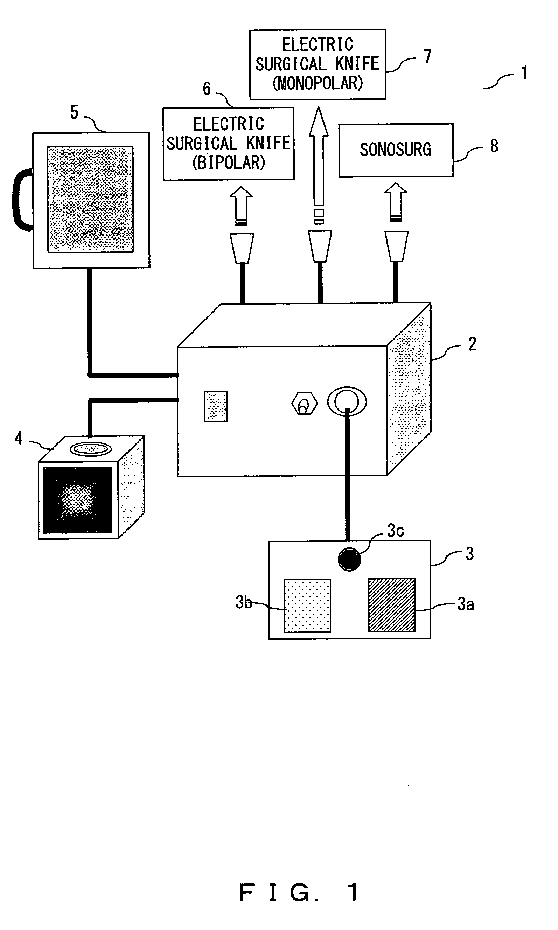

Fig. 1 illustrates a surgical operation system 1 in the present embodiment. The surgical operation system 1 has, for example, a controller 2, a footswitch 3, a speaker 4, a display panel 5, an electric surgical knife (bipolar) 6, an electric surgical knife (monopolar) 7, a SonoSurg (ultrasonic cutting and coagulating device) 8, and so on.

The footswitch 3 has two output pedals 3a, 3b and a switching button 3c. The footswitch 3 is used for the switch, by means of the switching button 3c of the footswitch 3, to a medical device that the operator wants to use, selected from connected medical devices, and for setting the output of the medical device by means of the output pedals 3a, 3b of the footswitch 3.

The controller 2 switches the medical devices to be operated and controls the outputs of the medical devices, on the basis of an instruction signal generated by the holding down of the switching button 3c and the output pedals 3a, 3b.

The speaker 4 is for providing voice guidance for the name or the type of the output of the selected medical device to be operated, in accordance with the operation detail of the footswitch 3. The display panel 5 is a touch panel for registering the setting of the medical devices in advance and for selecting the medical devices. The display panel 5 displays a menu screen, a medical device registration screen, and the selected mode.

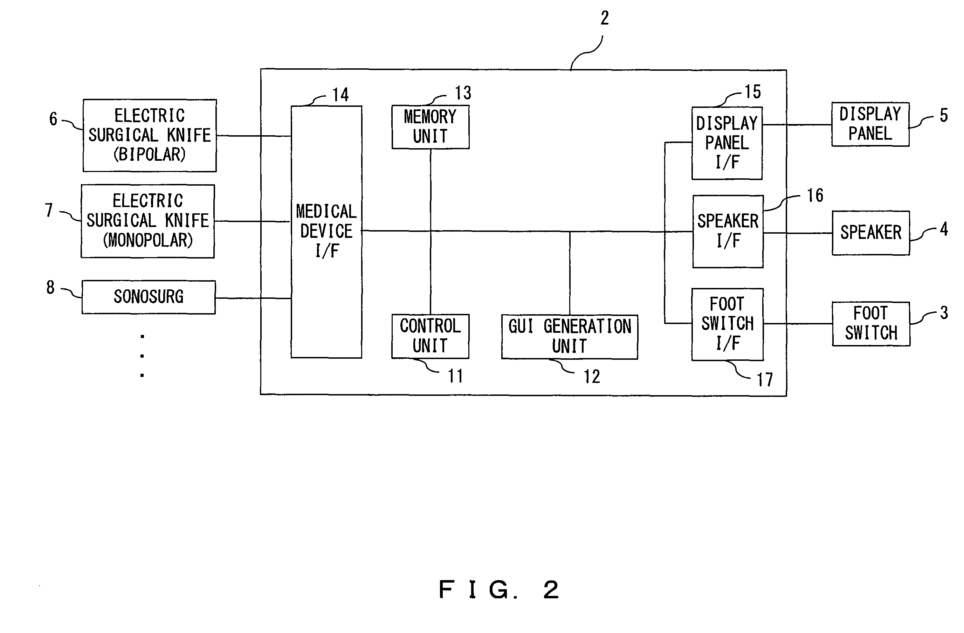

Fig. 2 illustrates the outline of the internal configuration of the controller 2 in the present embodiment. A footswitch 3, a speaker 4, a display panel 5, an electric surgical knife (bipolar) 6, an electric surgical knife (monopolar) 7, a SonoSurg 8, and so on are connected to the controller 2.

The controller 2 includes a control unit 11, a GUI generation unit 12, a memory unit 13, a medical device interface (hereinafter, an interface is referred to as I/F) 14, a display panel I/F 15, a speaker I/F 16, and a footswitch I/F 17.

The medical device I/F 14 is an interface for communicating with medical devices including, for example, the electric surgical knife (bipolar) 6, the electric surgical knife (monopolar) 7, the SonoSurg 8, a pneumoperitoneum device, a pump, and so on.

The memory unit 13 includes memories such as a RAM (Random Access Memory), a ROM (Read Only Memory) used for various processes and a hard disk drive (HDD) storing a large amount of data.

The control unit 11 includes a CPU and the like that reads in and executes various processing programs stored in the memory unit 13. The control unit 11 controls various devices constituting the controller 2, as well as the communication between the controller 2 and the connected medical devices.

The GUI generation unit 12 performs various image processing and composes an operation screen to be displayed on the display panel 5, on the basis of the control performed by the control unit 11. The screens described later are generated by the GUI generation unit 12.

The display panel I/F 15 is an interface for outputting GUI image data generated by the GUI generation unit 12 to the display panel 5, and for receiving an operation signal from the display panel 5.

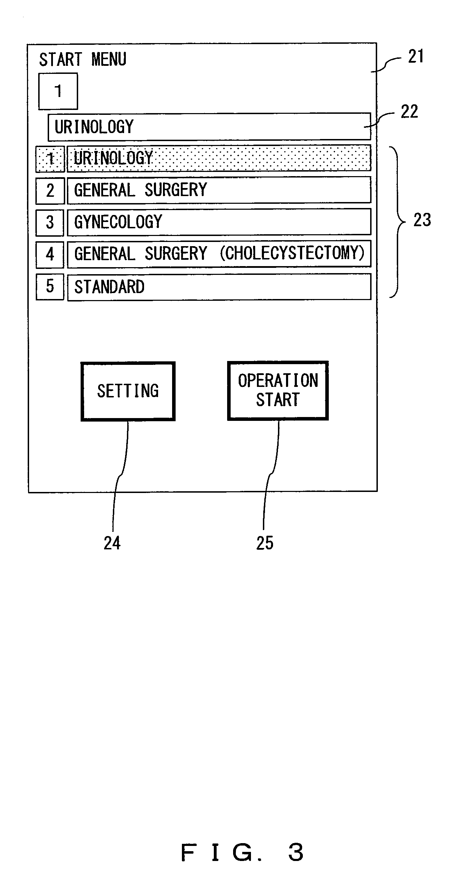

Fig. 3 illustrates a start menu screen 21 displayed on the display panel 5 in the present embodiment. The start menu screen 21 is displayed when the operator inputs an operator ID and a password on a login screen (not shown in the drawing). The start menu screen 21 has a selected course display space 22, a course list 23, a setting button 24, and an operation start button 25.

On the display panel 5, a predetermined course can be selected from courses displayed on the course list 23, including "1: URINOLOGY", "2: GENERAL SURGERY", "3: GYNECOLOGY", "4: GENERAL SURGERY (CHOLECYSTECTOMY)", "STANDARD", and so on. The course selected from the course list 23 is displayed in the selected course display space 22. In Fig. 3, "1: URINOLOGY" is selected from the course list 23 and displayed on the selected course display space 22.

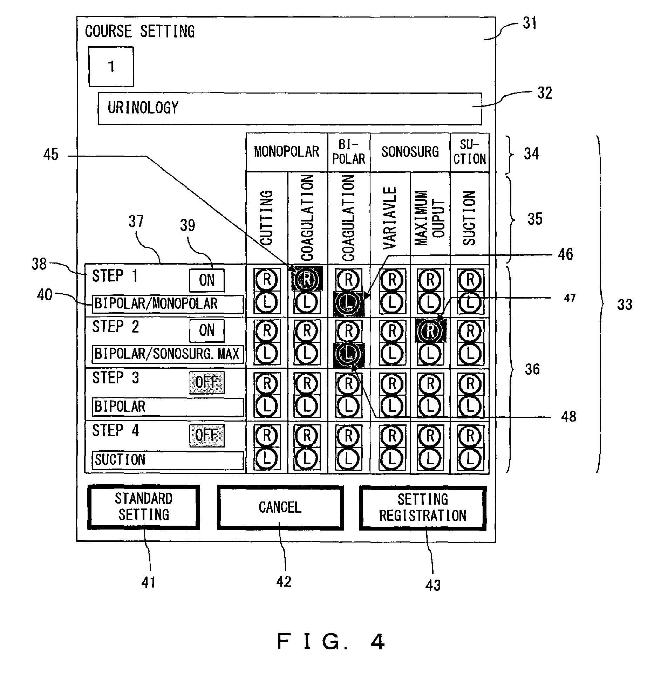

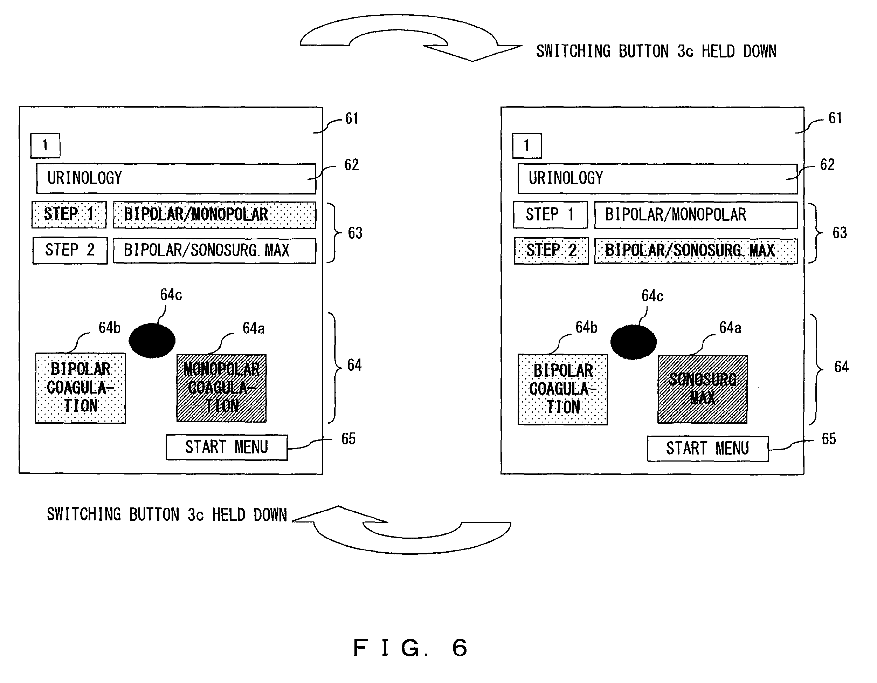

When the setting button 24 is held down after the course is selected from the course list 23, a shift to a course setting screen (Fig. 4) for performing detailed setting about the selected course is performed. When the operation start button 25 is held down, the screen shifts to the one in Fig. 6, and the mode shifts to the operation mode.

Fig. 4 illustrates a course setting screen 31 displayed on the display panel 5 in the present embodiment. The course setting screen 31 is composed of a setting course name display space 32, a footswitch function assignment setting space 33, a standard setting button 41, a cancel button 42, and a setting registration button 43.

The setting course name display space 32 displays the course name selected from the course list 23 on the start menu screen 21. The footswitch function assignment setting space 33 can be set for the course displayed in the setting course name display space 32.

In the footswitch function assignment setting space 33, any medical devices and their outputs that the operator wants to use may be assigned to the left and right pedals 3a, 3b, for each switching step.

The footswitch function assignment setting space 33 is composed of a medical device name display space 34, an output name display space 35, a pedal setting space 36, and a step setting space 37. The medical device name display space 34 displays the medical devices connected to the controller 2. The output name display space 35 displays the selectable output names of each device displayed in the medical device name display space 34. In the pedal setting space 36, the functions displayed in the outpour name display space 35 can be assigned to the left (L) /right (R) pedals, for each step.

In the step setting space 37, for each step that is switched each time the switching button 3c of the footswitch 3 is held down, the control corresponding to the operation of the pedals 3a, 3b of the footswitch 3 can be assigned. The step setting space 37 is composed of a step No. space 38, an ON/OFF display space 39, and a step name display space 40.

The step No. space 38 displays the step numbers. In Fig. 4, the setting for steps 1 through 4 may be performed.

The ON/OFF display space 39 displays "ON" when the step is valid (in other words, when the switch to the step can be performed using the footswitch), and displays "OFF" when the step is invalid (in other words, when the switch to the step cannot be performed using the footswitch). Specifically, "ON" is displayed when the setting of the pedals has been done for the step in the pedal setting space 36.

The step name display space 40 displays names based on the medical devices and their outputs assigned to the left and right pedals in the pedal setting space 36.

For example, in order to assign the output "coagulation" of the medical device "monopolar" to the right pedal 3a in STEP 1, "R" indicated by a numeral 45 is selected. In addition, in order to assign the output "coagulation" of the medical device "bipolar" to the left pedal 3b in STEP 1, "L" indicated by a numeral 46 is selected. Then, the step name display space 40 displays the device names "BIPOLAR/MONOPOLAR" assigned to the left pedal/right pedal, and the ON/OFF display space 39 displays "ON".

For example, in order to assign the output "maximum output" of the medical device "SonoSurg" to the right pedal 3a in STEP 2, "R" indicated by a numeral 47 is selected. In addition, in order to assign the output "coagulation" of the medical device "bipolar" to the left pedal 3b in STEP 2, "L" indicated by a numeral 48 is selected in step 2. Then, the step name display space 40 displays the device and output names "BIPOLAR/SONOSURG.MAX" assigned to the left pedal/right pedal, and the ON/OFF display space 39 displays "ON".

When the standard setting button 41 is held down, the setting in the footswitch function assignment setting space 33 becomes the default setting. When the cancel button 42 is held down, the screen returns to the previous screen, without registering the setting in the footswitch function assignment setting space 33 in the controller 2. When the setting registration button 43 is held down, the setting in the footswitch function assignment setting space 33 is registered in the controller 2, and the screen returns to the previous screen.

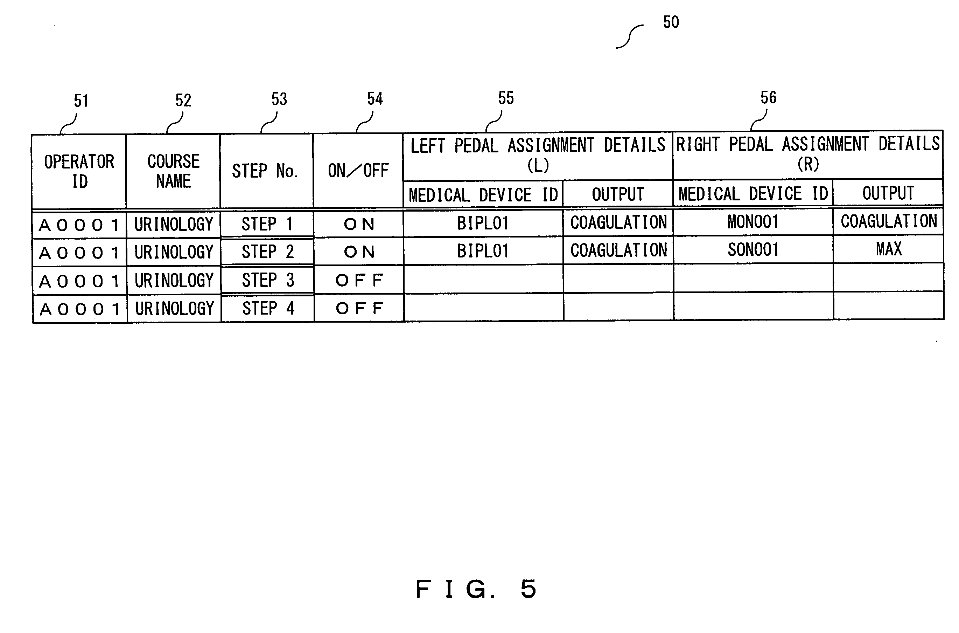

Fig. 5 illustrates the footswitch function assignment setting table 50 in the present embodiment. The footswitch function assignment setting table 50 is stored in the memory unit 13 of the controller 2. When the setting registration button 43 in the course setting screen 31 is held down on the display panel 5, the setting details of the steps set in the footswitch function assignment setting space 33 are registered in the footswitch function assignment setting table 50.

The footswitch function assignment setting table 50 is composed of data items including "operator ID" 51, "step No." 52, "course name" 53, "ON/OFF" 54, "left pedal assignment details (L)" 55, and "right pedal assignment details (R)" 56.

For example, when an operator having an operator ID "A0001" registers the setting details in Fig. 4, four records corresponding to STEP 1 through STEP 4 are generated. Then, "A0001" is stored in the "operator ID" 51; "Urinology" is stored in the "course name" 52; step numbers STEP 1 through STEP 4 are respectively stored in the "step No." 53 of each record; "ON/OFF" displayed in the ON/OFF display space 39 is stored in the "ON/OFF" 54. Each of the "left pedal assignment details (L)" 55 and "right pedal assignment details (R)" 56 is composed of data items "medical device ID" and "output", and stores details set in the pedal setting space 36.

Thus, the course can be registered for each operator and procedure, assigning given device/output to be used to the left and right pedals. Next, the usage of the footswitch after the course registration is described.

Fig. 6 illustrates a step switching screen 61 displayed on the display panel 5 in the present embodiment. The step switching screen 61 is displayed when the operation start button 25 in the start menu screen 21 is held down. The step switching screen 61 displays the medical devices and contents of outputs selected from the medical devices connected to the controller 2 that the operator wants to use by switching by means of the footswitch 3.

The step switching screen 61 is composed of a selected course display space 62, a step-in-use switching list 63, a footswitch setting detail display unit 64, and a start menu button 65. The selected course display space 62 displays the "course name" displayed in the selected course display space 22 in the start menu screen 21. The screen returns to the start menu screen 21 when the start menu button 65 is held down.

The step-in-use switching list 63 displays step setting information corresponding to the records of which "ON/OFF" 54 in the footswitch function assignment setting table 50 is ON. Specifically, first, when the screen switches from the start menu screen 21 to the step switching screen 61, the control unit 11 reads out the footswitch function assignment setting table 50 from the memory unit 13. Then, the control unit 11 extracts predetermined records with the "operator ID" of the operator who has logged in and the "course name" displayed in the selected course display space 22 in the start menu screen 21 as the key. Then, the control unit 11 obtains, from the extracted records, only the records of which "ON/OFF" 54 is "ON", and makes the step-in-use switching list 63 display, as the step setting information, the switching information corresponding to the obtained records.

The step setting information selected in the step-in-use switching list 63 can be selected sequentially, every time the switching button 3c of the footswitch is held down. The selected step setting information is displayed with emphasis.

The footswitch setting detail display unit 64 displays the step setting information selected in the step-in-use switching list 63 as an image assigned to each pedal of the footswitch 3, so as to facilitate the visual perception of the operator. The area indicated by a numeral 64a displays the device and output assigned to the right pedal. The area indicated by a numeral 64b displays the device and output assigned to the left pedal. The figure indicated by a numeral 64c represents the switching button 3c.

In the case of Fig. 6, the switch is performed in the order of step1, step 2, step 1 ... every time the switching button 3c of the footswitch 3 is held down, and the display on the footswitch setting detail display unit 64 changes accordingly.

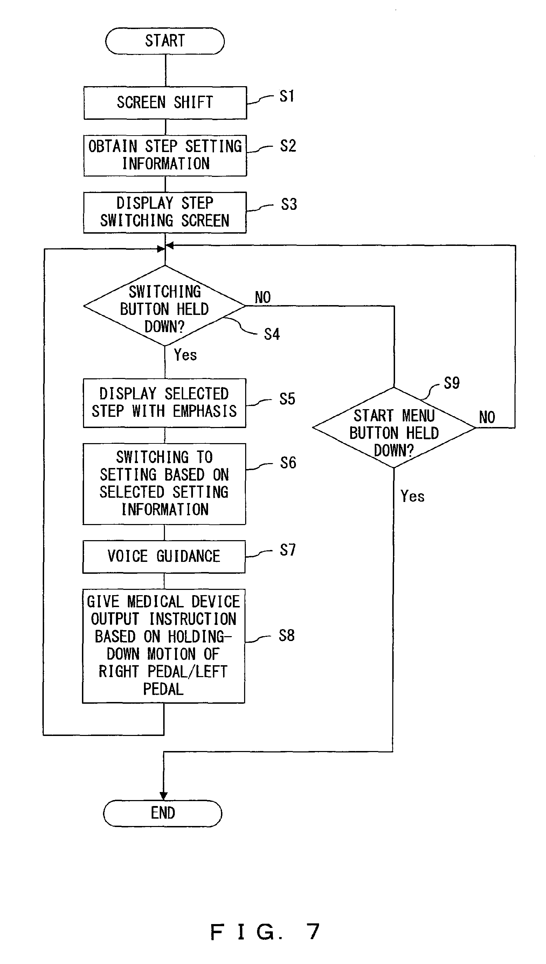

Fig. 7 illustrates a process flow of a step switching operation in the present embodiment. When the operation start button 25 on the start menu screen 21 is held down, the control unit 11 starts the process of shifting to the step switching screen 61 (S1).

The control unit 11 reads out the footswitch function assignment setting table 50 from the memory unit 13. Then, the control unit 11 extracts predetermined records with the "operator ID" of the operator who has logged in and the "course name" displayed in the selected course display space 22 in the start menu screen 21 as the key. Then, the control unit 11 obtains, from the extracted records, the records of which "ON/OFF" 54 is "ON" as the step setting information (S2).

Then, the control unit 11 makes the GUI generation unit 12 display the step switching screen 61 with the step-in-use switching list 63 set with the step setting information (S3).

When the switching button 3c of the footswitch 3 is held down (S4), a switching signal is transmitted to the control unit 11. The control unit 11 receives the switching signal and instructs the GUI generation unit 12 to perform the switching of the step-in-use. Having received the instruction from the control unit 11, the GUI generation unit 12 displays, with emphasis, the record subsequent to the record that is currently displayed with emphasis in the step-in-use switching list 63 (S5).

Then, the control unit 11 switches the setting to the setting based on the selected step setting information (S6). Specifically, the control unit 11 reads in, as new setting information, the information held in "left pedal assignment details" 55 and "right pedal assignment details" 56 corresponding to the selected step setting information and performs the setting. The newly set details are output with voice from the speaker 4 to provide voice guidance (S7).

The control unit 11 gives the medical devices an output instruction in accordance with the holding-down motion of the right pedal 3a/left pedal 3b of the footswitch 3 (S8). A case assuming left pedal assignment details "bipolar: coagulation" and right pedal assignment details "SonoSurg: MAX" is explained for example. When the left pedal 3b is held down, an instruction signal is transmitted to the control unit 11. Having received the instruction signal, the control unit 11 instructs the electric surgical knife (bipolar) 6 to perform output for coagulation. Meanwhile, when the right pedal 3a is held down, the control unit 11 instructs the SonoSurg 8 to perform output at the maximum level. Each medical device operates in accordance with the instruction.

The processes in S4 through S8 are repeated every time the switching button 3c of the footswitch 3 is held down. When the start menu button 65 is held down ("Yes" in S9), the screen returns to the previous screen, and the flow is terminated.

Thus, according to the present embodiment, the footswitch has two output pedals and one switching unit. The outputs of medical devices can be assigned to the output pedals of the footswitch in advance, and a plurality of steps for the assignment can be registered. The steps can be switched by means of the switching unit of the footswitch in accordance with the registered details. The course composed of the assignment for the outpour pedals and the number of steps can be set for each operator in advance.

While an example of assigning the output control of medical devices to the left and right pedals is described with respect to the present embodiment, this is not a limitation, and the output control of medical devices may be assigned to only one of the pedals.

According to the present embodiment, the outputs of a plurality of medical devices such as electric surgical knives, an ultrasonic cutting and coagulating device, a pneumoperitoneum device, a pump, and so on can be controlled with a single footswitch. In addition, the output setting of the medical devices can selected with a single footswitch for each operator. Therefore, the output operations required for the operator can be switched in advance with minimum necessary operations.

### <Second embodiment>

With respect to the present embodiment, a system in which the usage history of medical devices can be recorded by recording the operation data of the footswitch in the first embodiment is explained. For the same constituent elements as the ones in the first embodiment, the same numerals are assigned and the explanation for them is omitted.

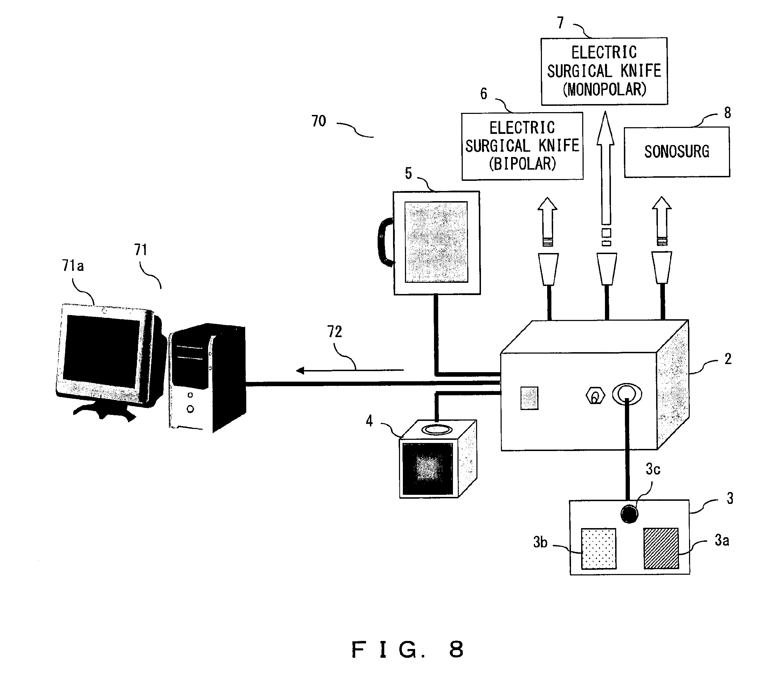

Fig. 8 illustrates the overall configuration of a surgical operation system 70 in the present embodiment. In Fig. 8, a computer 71 is added to the surgical operation system 1 in Fig. 1. The operation history of a foot pedal 3 is stored in the memory unit 13 as footswitch usage history information 72.

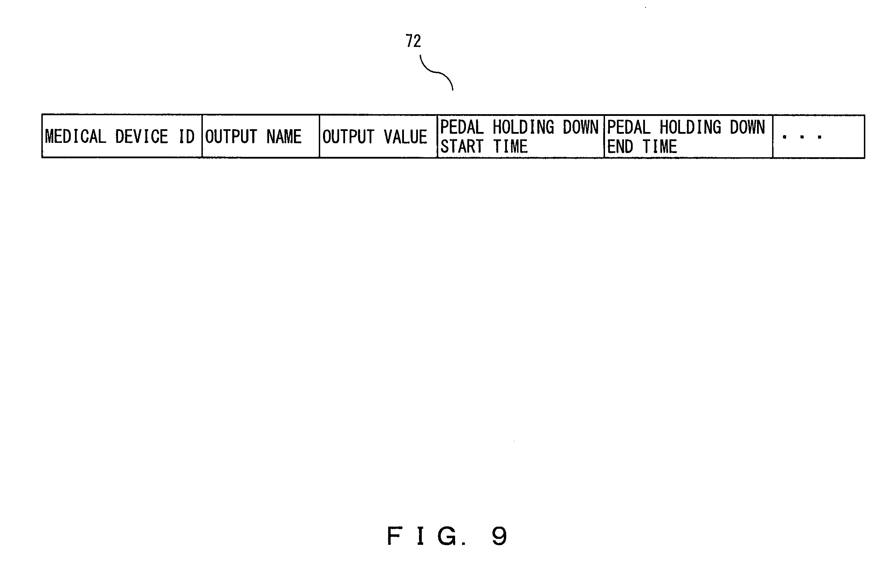

Fig. 9 illustrates an example of footswitch usage history information 72 in the present embodiment. The footswitch usage history information 72 includes data items such as "medical device ID", "output name", "output value", "pedal holding down start time", "pedal holding down end time", and so on. The "medical device ID" is an ID for identifying a medical device. The "output name" is a name specifying an output of a medical device assigned to a pedal of the footswitch 3. The "output value" is a value of an output. The "pedal holding down start time" and "pedal holding down end time" are the times on which the holding down of the pedal of the footswitch 3 is started, and the time on which the holding down is terminated, respectively.

The control unit 11 obtains the "pedal holding down start time" and "pedal holding down end time" of the output pedals 3a, 3b on the basis of instruction signals from the respective pedals. The control unit 11 further associates the "pedal holding down start time" and "pedal holding down end time" with the currently-used "medical device ID", "output name", "output value" and stores them in the memory unit 13 as the footswitch usage history information 72.

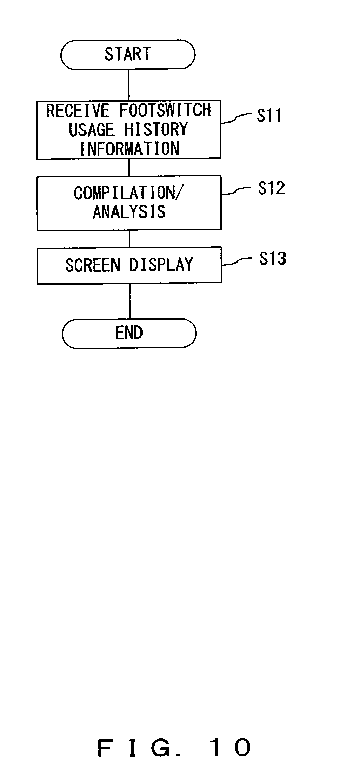

Fig. 10 illustrates processes performed by the computer 71 in the present embodiment. When the computer 71 issues a transmission request for the footswitch usage history information 72, the controller 2 obtains the footswitch usage history information 72 from the memory unit 13, and transmits it to the computer 71.

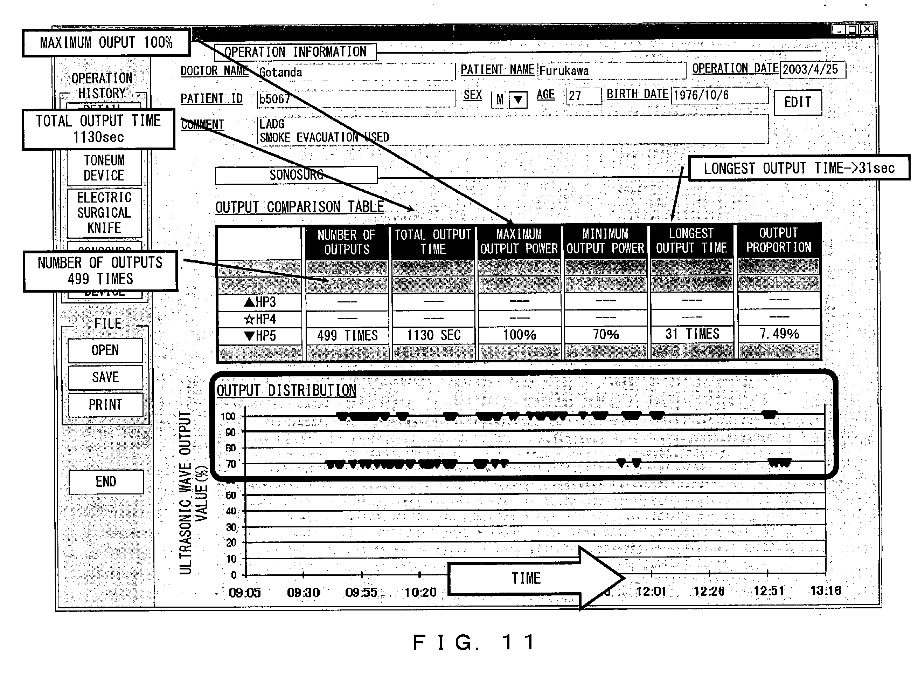

Upon receiving the footswitch usage history information 72 (S11), the computer 71 performs analysis to compile the number of output from them medical device, the total output time, the maximum output power, the minimum output power, the longest output time, the output proportion and the like, and to calculate the output distribution (S12). The computer 71 displays the result of the analysis/compilation on the display 71a as illustrated in Fig. 11 (S13).

Fig. 11 illustrates an example of a medical device operation history checking screen in the present embodiment. A medical device operation history checking screen generated by the computer 71 on the basis of the footswitch usage history information 72 about the SonoSurg is illustrated in Fig. 11. In Fig. 11, according to the analysis/compilation performed for the footswitch usage history information 72, the values "output time: 499 times", "total output time: 1130 seconds", "maximum output power: 100%", "minimum output power: 70%", "longest output time: 31 seconds", "output proportion; 7.49%" are displayed with respect to the output "HP5" of the SonoSurg, as well as the output distribution chart.

Thus, according to the present embodiment, the footswitch usage history information of the footswitch can be recorded and output from the controller to the computer. The computer 71 is able to perform analysis/compilation of the footswitch usage history information. Meanwhile, the display style on the display is not limited as descried in the present embodiment, and various known display styles may be used depending on the purpose.

### <Third embodiment>

The present embodiment makes it possible to automatically recognize the medical device that the operator is actually holding, and to assign an output registered in advance to each pedal of the footswitch on the basis of the result of the recognition. For the same constituent elements as the ones in the first embodiment, the same numerals are assigned and the explanation for them is omitted.

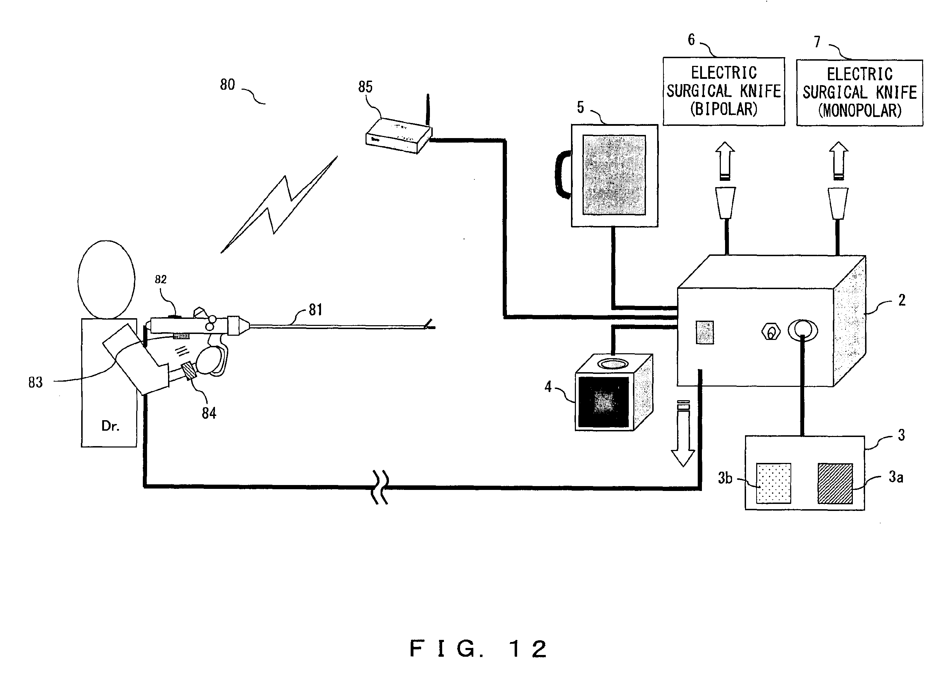

Fig. 12 illustrates an overall configuration of a surgical operation system 80 in the present embodiment. The operator is wearing a wristband 84 with an embedded IC tag on the right wrist, and is holding a SonoSurg probe 81 with the right hand. The SonoSurg probe 81 is equipped with a reader 83 for reading an IC tag and a transmission unit 82 for transmitting data to a reception terminal 85. While the footswitch 3 does not have a switching button 3c in Fig. 12, a footswitch 3 with a switching button 3c may also be used.

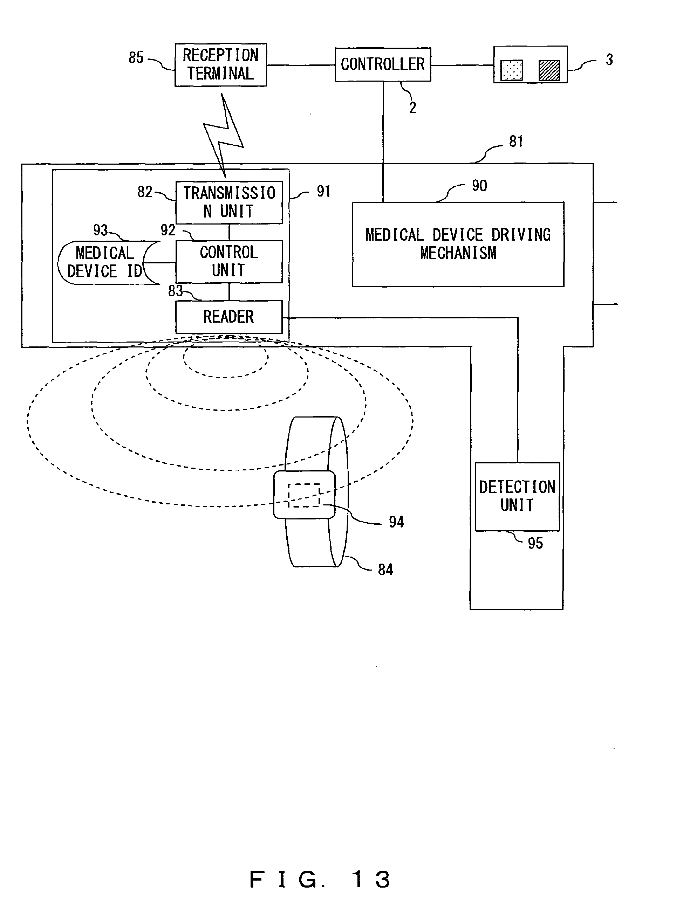

Fig. 13 illustrates the outline of an IC tag reading and transmitting mechanism in the SonoSurg probe 81 in the present embodiment. An IC tag reading and transmitting mechanism 91 is independent from a medical device driving mechanism 90 related to operation and control of medical devices, and may either be a built-in type or an external type.

The IC tag reading and transmitting mechanism is composed of an IC tag reading and transmitting unit 91 and a detection unit 95. The detection unit 95 is a sensor (a pressure sensor, a thermal sensor and the like) or a switch that determines whether or not the operator has held a medical device or not. The IC tag reading and transmitting unit 91 is composed of a reader 83, a control unit 92, a memory unit 93, and a transmitting unit 82.

The memory unit 93 stores medical device IDs. The reader 83 is driven on the basis of the result of detection performed by the detection unit 95, and reads out information stored in an IC tag 94 in a wristband 84. The reader 83 reads information within a close range, i.e., within a range in which the IC tag 94 of the wristband 84 worn by an operator holding a medical device can be read.

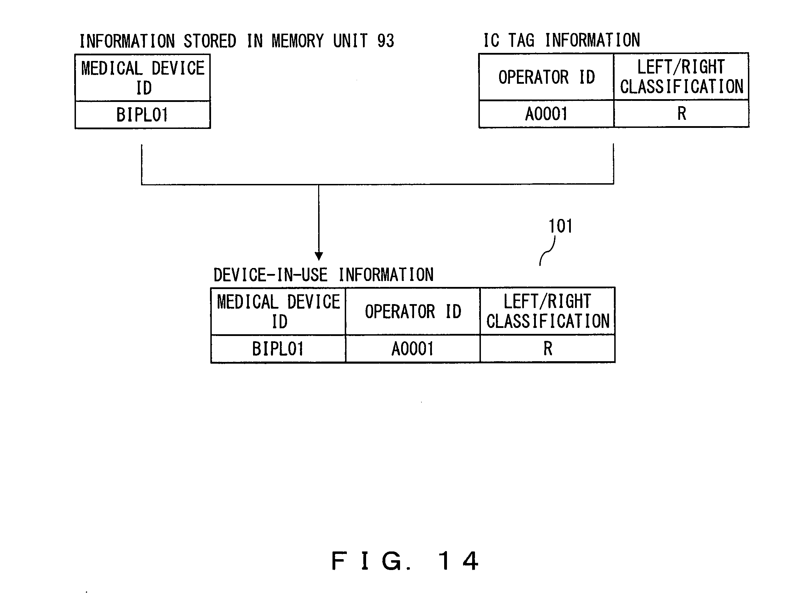

Fig. 14 illustrates the transition of data from the acquisition to the transmission, to a reception terminal 85, of IC tag information by the IC tag reading and transmitting mechanism 91 in the present embodiment. The IC tag 94 stores "operator ID" and "left/right classification" as IC tag information.

The "operator ID" stores the operator ID of the person who is actually wearing the wristband 84. The "left/right classification" stores information about on which arm the person is wearing the wristband. Therefore, when the wristband 84 is worn on the right arm, "R" is stored in the left/right classification", when the wristband 84 is worn on the left arm, "L" is stored in the left/right classification". The wristband 84 may be worn either on one arm or on both arms. Even when the wristbands are worn on both arms and each hand holds a medical device, the reader 83 mounted on the medical device held with the left arm does not read the IC tag information of the wristband on the right arm, due to the limitation of the reading range of the reader 83 described above.

The control unit 92 generates device-in-use information 101 that is information in which the IC tag information and the medical device ID are associated. The device-in-use information 101 is transmitted to the reception terminal 85 by the transmission unit 82. The device-in-use information 101 received by the reception terminal 85 is transmitted to the controller 2.

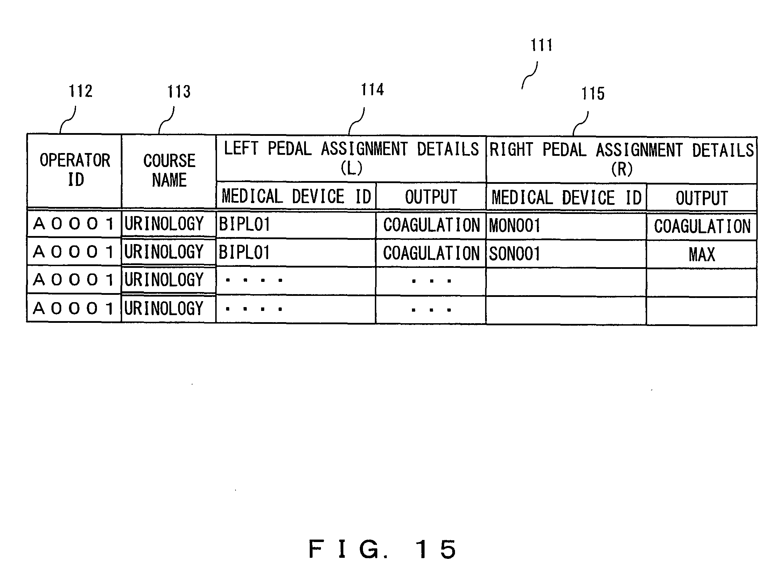

Fig. 15 illustrates a footswitch function assignment setting table 111 in the present embodiment. In Fig. 15, in the same way as in the first embodiment, the combination of functions to be assigned to the left and right pedals 3a, 3b is registered in advance using the display panel 5, and stored in the memory unit 13.

The footswitch function assignment setting table 111 is composed of "operator ID" 112, "course name" 113 "left pedal assignment details (L) "114, and "right pedal assignment details (R)" 115. The "left pedal assignment details (L) " 114 and "right pedal assignment details (R) " 115 are respectively composed of "medical device ID" and "output". The "operator ID" 112, "course name", "left pedal assignment details (L) " 114, and "right pedal assignment details (R) " 115 correspond to the "operator ID" 56, "course name" 53, "left pedal assignment details (L) " 55, and "right pedal assignment details (R)" 56, respectively.

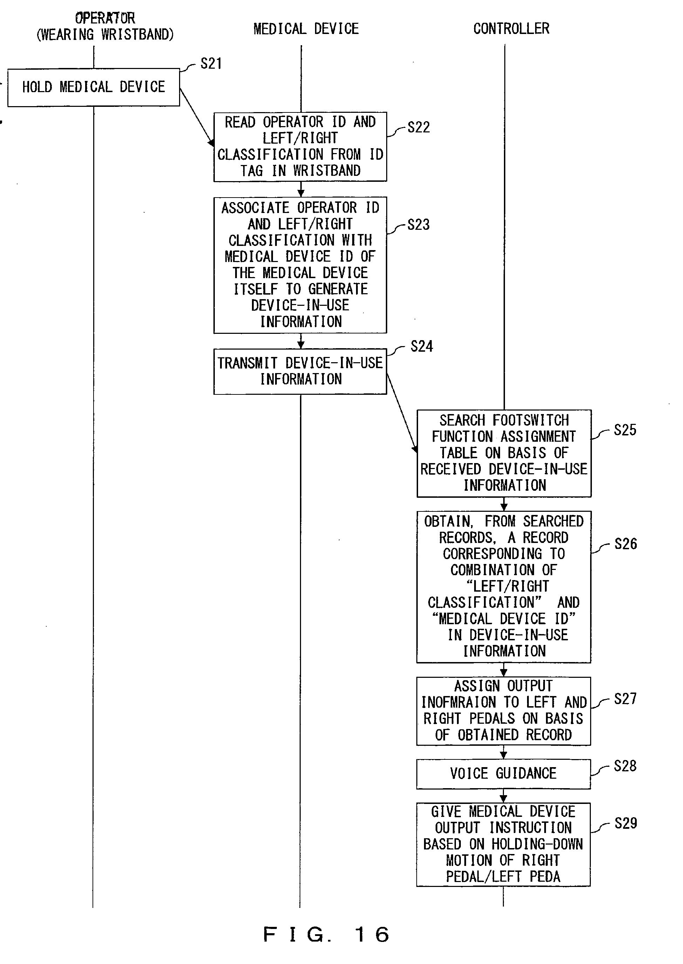

Fig. 16 illustrates a flow of a series of processes in the present embodiment. Fig. 16 is explained with reference to Fig. 12 through Fig. 15. First, an operator wearing wristbands 84 on left and right arms holds a given medical device (or a medical tool) in accordance with the operation course (S21). The detection unit 95 then detects the holding motion done by the operator, and transmits a detection signal to the reader 83.

Upon receiving the detection signal from the detection unit 95, the reader 83 reads out IC tag information from the IC tag 94 in the wristband 84 (S22). Specifically, the reader 83 transmits a predetermined electric wave.

Upon receiving the electric signal from the reader 83, the IC tag 94 is driven and transmits IC tag information that has been stored in advance. The reader 83 receives the transmitted IC tag information. Then, the control unit 92 performs a decode process for the transmitted IC tag information, and obtains the "operator ID" and "left/right classification" as IC tag information.

Next, the control unit 92 reads out the "medical device ID" from the memory unit 93, and associates the "medical device ID" with the "operator ID" and the "left/right classification" to generate device-in-use information 101 (S23). The device-in-use information 101 is transmitted to the reception terminal 85 by the transmission unit 82 (S24). The device-in-use information for the right hand and device-in-use information for the left hand are transmitted, in the present embodiment.

Upon receiving the device-in-use information 101, the reception terminal 85 transmits it to the controller 2. Upon receiving the device-in-use information 101, the controller 2 searches the footswitch function assignment setting table 111 with the "operator ID" in the device-in-use information 101 and the "course name" that has been identified in advance as the key (S25).

The controller 2 obtains, on the basis of the "left/right classification" and the "medical device ID" in the device-in-use information, a record that corresponds to the combination, from the searched records (S26). For example, when two pieces of device-in-use information "BIPL01, A0001, L" and "SONO01, A0001, R" have been obtained, it indicates that the medical device "BIPL01" is held with the "left (L) " hand, and "SONO01" is held with the "right" hand. Therefore, the controller 2 obtains the record (in the case of Fig. 15, the second record from the top) that corresponds to the combination.

The controller 2 assigns output information to the left and right pedals 3a and 3b on the oasis of the obtained record (S27). In other words, the controller 2 sets the "output" information held in the "left pedal assignment details (L) " 114 and "right pedal assignment details(R)" 115 of the obtained record to the corresponding pedals. The newly set details are output with voice from the speaker 4 (S28).

In this condition, the controller 2 gives the medical device an output instruction in accordance with the holding-down motion of the right pedal 3a/left pedal 3b of the footswitch 3 (S29) . A case assuming left pedal assignment details "BIPL01 (bipolar) : coagulation" and right pedal assignment details "SONO01(SonoSurg): MAX" is explained for example. When the left pedal 3b is held down, the controller 2 instructs the electric surgical knife (bipolar) 6 to perform output for coagulation. Meanwhile, when the right pedal 3a is held down, the control unit 11 instructs the SonoSurg 8 to perform output at the maximum level. The respective medical devices operate in accordance with the instructions.

While the IC tag is provided on the operator side and the reader is provided on the medical device side according to the present embodiment, this is not a limitation. For example, a wristband equipped with a reader having a transmission unit may be provided on the operator side, and an IC tag may be provided on the medical device side. In addition, the transmission unit 82 and the reception terminal 85 may be connected by wireline connection. In addition, while an example of assigning the output control of medical devices to the left and right pedals is described with respect to the present embodiment, this is not a limitation, and the output control of medical devices may be assigned to only one of the pedals.

According to the present embodiment, no switching by means of a switching button of the footswitch is required, and even if the operator does not voluntarily select the combination of medical devices to be used, the automatic recognition can be performed only with the actual motion of the operator holding the medical device to be used. Therefore, the operator can start the usage of the medical device immediately.

As described above, an operation system according to the present invention includes a controller (2) controlling a plurality of medical devices and a footswitch (3) transmitting an instruction signal to the controller. The medical device is, for example, an electric surgical knife device, an ultrasonic coagulating and cutting device, a pneumoperitoneum device or a pump and the like.

The footswitch (3) has a selection unit (3c) and a first and second pedals (3a, 3b). The selection unit (3c) is capable of selecting, from the medical devices, a medical device to be used and an output of the medical device. The first and second pedals are capable of instructing the selected medical device to perform the output.

The controller (2) has a memory unit (13) and a control unit (11). The memory unit stores output pedal setting information that includes operator identification information identifying an operator, first pedal setting information and second pedal setting information. The first pedal setting information is composed of device identification information identifying the medical device corresponding to the first pedal and information about an output of the medical device corresponding to the first pedal. The second pedal setting information is composed of device identification information identifying the medical device corresponding to the second pedal and information about an output of the medical device corresponding to the second pedal.

The control unit (11) controls, when the selection unit selects given output pedal setting information and the respective pedals are held down, the respective medical devices on the basis of the first and second pedal setting information in the output pedal setting information.

According to the present invention, by adopting a configuration as described above, the output operations of medical devices required by each operator can be switched by minimum necessary switching operations. In other words, medical devices to be used can be switched and their outputs can be adjusted using a footswitch having one switching button and two pedals. Accordingly, output operations of medical devices required by each operator can be switched by minimum necessary switching operations.

In addition, the control unit (11) is capable generating and storing, in the memory unit (13), usage histories of the first and second pedals on the basis of instruction signals from the first and second pedals. By adopting such a configuration, the usage history of medical devices can be recorded by recording the operation data of the footswitch.

The operation system further has a display unit (71a) displaying an output state of the medical device on the basis of the usage histories of the first and second pedals. By adopting such a configuration, the usage histories can be compiled in accordance with the purpose, and can be displayed in a display format such as a graph, matrix and the like that facilitates visual perception.

The operation system may further comprise a wristband (84). The wristband (84) is to be worn on each arm of the operator. The wristband has an IC tag (94) storing operator identification information of the operator and left/right classification information determining on which arm the wristband is worn.

The medical device has a reading unit (83), a device-in-use information generation unit (92), and a transmission unit (82).

The reading unit (83) reads the IC tag (94) to obtain the operator identification information and the left/right classification information. The device-in-use information generation unit (92) generates device-in-use information in which the operator identification information and the left/right classification information are associated with device identification information of the medical device itself. The transmission unit (82) transmits the device-in-use information.

By adopting a configuration as described above, IC tag information of a wristband of the operator can be obtained.

When the controller receives the device-in-use information, the control unit searches the output pedal setting information from the memory unit on the basis of the operator ID information in the device-in-use information. The control unit then extracts, from the output pedal setting information, the output pedal setting information composed of a combination of first pedal setting information and second pedal information corresponding to the left/right classification information and the medical device identification information in the device-in-use information. The control unit then controls the respective medical devices on the basis of the extracted first and second pedal setting information, in accordance with holding-down states of the respective pedals.

By adopting a configuration as described above, the automatic recognition can be performed to determine with which hand a medical device is held, only with the actual motion of holding the medical device to be used, and the output of the medical device can be assigned to each pedal.

Meanwhile, the present invention is not limited to the embodiments described above, and various configurations or embodiments may be adopted without departing from the scope of the present invention.

## Claims

1. An operation system comprising:
a controller (2) controlling a plurality of medical devices and
a footswitch (3) transmitting an instruction signal to the controller,
wherein
the footswitch (3) comprises:
a selection unit (3c) capable of selecting, from the medical devices, a medical device to be used and an output of the medical device; and
a first and second pedals (3a, 3b) capable of instructing the selected medical device to perform the output, and
the controller (2) comprises:
a memory unit (13) storing output pedal setting information comprising operator identification information identifying an operator; first pedal setting information comprising device identification information identifying the medical device corresponding to the first pedal and information about an output of the medical device corresponding to the first pedal; and second pedal setting information comprising device identification information identifying the medical device corresponding to the second pedal and information about an output of the medical device corresponding to the second pedal; and
a control unit (11) controlling, when the selection unit selects given output pedal setting information and the respective pedals are held down, the respective medical devices on the basis of the first and second pedal setting information in the output pedal setting information.

2. The operation system according to claim 1, wherein
the control unit (11) generates and stores, in the memory unit, usage histories of the first and second pedals on the basis of instruction signals from the first and second pedals.

3. The operation system according to claim 2, further comprising
a display unit (71a) displaying an output state of the medical device on the basis of the usage histories of the first and second pedals.

4. The operation system according to claim 1, further comprising:
a wristband (84) worn on each arm of the operator, the wristband having an IC tag storing operator identification information of the operator and left/right classification information determining on which arm the wristband is worn, wherein
the medical device comprises:
a reading unit (83) reading the IC tag to obtain the operator identification information and the left/right classification information;
a device-in-use information generation unit (92) generating device-in-use information in which the operator identification information and the left/right classification information are associated with device identification information of the medical device itself; and
a transmission unit (82) transmitting the device-in-use information.

5. The operation system according to claim 4, wherein
when the controller (2) receives the device-in-use information,
the control unit (11) searches the output pedal setting information from the memory unit on the basis of the operator ID information in the device-in-use information; extracts, from the output pedal setting information, the output pedal setting information composed of a combination of first pedal setting information and second pedal information corresponding to the left/right classification information and the medical device identification information in the device-in-use information; and controls the respective medical devices on the basis of the extracted first and second pedal setting information, in accordance with holding-down states of the respective pedals.

6. The operation system according to claim 1, wherein
the medical device is an electric surgical knife device, an ultrasonic coagulating and cutting device, a pneumoperitoneum device or a pump.

7. An operation system comprising:
a plurality of medical devices;
a controller (2) controlling the medical devices;
a footswitch (3) having first and second pedals capable of instructing the medical devices to perform the output and transmitting an instruction signal to the controller in accordance with holding-down states of the respective pedals; and
a wristband (84) worn on each arm of the operator, the wristband having an IC tag storing operator identification information of the operator and left/right classification information determining on which arm the wristband is worn, wherein
the medical device comprises:
a reading unit (83) reading the IC rag to obtain the operator identification information and the left/right classification information;
a device-in-use information generation unit generating (92) device-in-use information in which the operator identification information and the left/right classification information are associated with device identification information of the medical device itself; and
a transmission unit (82) transmitting the device-in-use information, and
the controller (2) comprises:
a memory unit (13) storing output pedal setting information comprising operator identification information identifying an operator; first pedal setting information comprising device identification information identifying the medical device corresponding to the first pedal and information about an output of the medical device corresponding to the first pedal; and second pedal setting information comprising device identification information identifying the medical device corresponding to the second pedal and information about an output of the medical device corresponding to the second pedal; and
a control unit (11) searching the output pedal setting information from the memory unit on the basis of the operator ID information in the device-in-use information; extracting, from the output pedal setting information, the output pedal setting information composed of a combination of first pedal setting information and second pedal information corresponding to the left/right classification information and the medical device identification information in the device-in-use information; and controlling the respective medical devices on the basis of the extracted first and second pedal setting information, in accordance with holding-down states of the respective pedals.

8. The operation system according to claim 7, wherein the medical device further comprises:
a detection unit (95) detecting that the operator has held the medical device, and
the reading unit (83) reads out the operator identification information and the left/right classification information from the IC tag of the wristband, on the basis of a result of detection performed by the detection unit.
